# FASCICULE DE BREVET EUROPEEN

(11) **EP 2 675 527 B1**
(45) Date de publication et mention de la délivrance du brevet: **31.10.2018**
(21) Numéro de dépôt: 12703815.6
(22) Date de dépôt: 15.02.2012
(51) Int. Cl.: A61P 15/14, A61K 31/48, A61K 31/381, A61K 31/4045, A61K 31/428

(54) **COMPOSITION VETERINAIRE ANTIPROLACTINIQUE DESTINEE AUX RUMINANTS COMPRENANT LA CABERGOLINE**
VETERINÄRMEDIZINISCHE ANTI-PROLACTIN-ZUSAMMENSETZUNG FÜR WIEDERKÄUER, DIE CABERGOLIN ENTHÄLT
VETERINARY ANTI-PROLACTIN COMPOSITION FOR RUMINANTS COMPRISING CABERGOLIN

(30) Priorité: 15.02.2011 FR 1151243
(43) Date de publication de la demande: 25.12.2013
(73) Titulaire: Ceva Santé Animale, 33500 Libourne (FR)
(72) Inventeur: ISAKA, Naomi, F-33500 Libourne (FR); BERTAIM, Thierry, F-33200 Coutras (FR)
(74) Mandataire: Tezier Herman, Béatrice
(86) Numéro de dépôt international: PCT/EP2012/052553
(87) Numéro de publication internationale: WO 2012/110537

(56) Documents cités:
- WO-A2-2006/117784
- FR-A1- 2 936 710
- ANNEN E L ET AL: "Effect of Modified Dry Period Lengths and Bovine Somatotropin on Yield and Composition of Milk from Dairy Cows", JOURNAL OF DAIRY SCIENCE, AMERICAN DAIRY SCIENCE ASSOCIATION, US, vol. 87, no. 11, 1 novembre 2004 (2004-11-01), pages 3746-3761, XP026974749, ISSN: 0022-0302 [extrait le 2004-11-01]
- GULAYA M S ET AL: "Milk Production and Feed Intake of Holstein Cows Given Short (30-d) or Normal (60-d) Dry Periods", JOURNAL OF DAIRY SCIENCE, AMERICAN DAIRY SCIENCE ASSOCIATION, US, vol. 86, no. 6, 1 juin 2003 (2003-06-01), pages 2030-2038, XP009151128, ISSN: 0022-0302, DOI: 10.3168/JDS.S0022-0302(03)73792-8 [extrait le 2010-03-27]
- VELASCO J M ET AL: "Short-Day Photoperiod Increases Milk Yield in Cows with a Reduced Dry Period Length", JOURNAL OF DAIRY SCIENCE, AMERICAN DAIRY SCIENCE ASSOCIATION, US, vol. 91, no. 9, 1 septembre 2008 (2008-09-01), pages 3467-3473, XP026955019, ISSN: 0022-0302 [extrait le 2008-09-01]

## Description

La présente divulgation a pour objet l'utilisation d'une composition vétérinaire ainsi qu'une méthode destinées à prévenir et/ou à réduire les effets délétères qui sont traditionnellement observés lors de la mise en oeuvre d'une période sèche raccourcie chez les ruminants. Ceux-ci comprennent notamment des maladies métaboliques et des troubles de la reproduction à la reprise de la lactation. L'utilisation des compositions vétérinaires et selon l'invention n'affectent pas la capacité de production et/ou la qualité du lait du ruminant lors de la lactation suivante.

Chez les ruminants, la lactation dure de 5 à 20 mois en général. Elle est le plus souvent de 10 mois chez la vache laitière. Après cette période de lactation, on arrête en général brutalement la traite et le ruminant ne produit plus de lait jusqu'au vêlage, point de départ de la lactation suivante. Cette période entre l'arrêt de la traite et le vêlage, dite période sèche, est fixée généralement à environ 2 mois chez la vache laitière.

Bien qu'une période sèche soit nécessaire pour assurer une production laitière optimale lors de la lactation suivante, cette période sèche oblige à plusieurs changements et transitions alimentaires pour s'adapter aux besoins physiologiques d'un animal qui ne produit plus de lait. Ces changements alimentaires induisent de nombreux bouleversements et stress physiologiques chez le ruminant, tels que des perturbations d'équilibre des flores digestives, la baisse de la capacité d'ingestion et d'absorption des estomacs avec modifications de la taille du rumen et des villosités de la muqueuse ruminale. Finalement, cette période sèche aboutit à un métabolisme chez le ruminant très différent de celui observé pendant la lactation. Ainsi, lors de la reprise de la lactation après vêlage, le ruminant se retrouve dans un état d'inadaptation métabolique qui se traduit par un déficit énergétique, alors que les besoins pour la production laitière sont à nouveau maximaux.

Par ailleurs, les changements et les transitions alimentaires qu'impose une période sèche longue, rendent plus complexes la gestion des programmes alimentaires au niveau du troupeau. Une simplification de ces programmes, du fait d'un raccourcissement de la période sèche, faciliterait la conduite du troupeau pour l'éleveur.

Des tentatives de réduction de la durée de la période sèche ont été faites dans le passé afin de diminuer les conséquences négatives décrites précédemment. Ainsi chez la vache, des périodes sèches d'1 mois au lieu des 2 mois habituels ont été essayées. Il s'est avéré néanmoins que la pratique des périodes sèches raccourcies aggravait en fait les déséquilibres métaboliques des ruminants et se traduisait par un risque accru de survenue de maladies métaboliques et de troubles de la reproduction dans les premiers mois de la lactation suivante. En plus de ce préjudice important pour le ruminant, ces pratiques entraînent généralement une baisse de la production laitière lors de lactation.

La demande de brevet WO2006/117784 décrit ainsi l'utilisation d'un dérivé de la caséine pour réduire la période sèche des ruminants.

En médecine vétérinaire, des compositions à base d'inhibiteur de la prolactine telles que le Galastop® (cabergoline, Ceva Santé Animale) peuvent être administrées en tant que traitements des lactations de pseudo-gestations chez les chiennes. Elles sont aussi prescrites suite à un sevrage précoce, aux avortements, ou lors du retrait immédiat de la portée après la parturition afin de stopper la montée de lait et l'allaitement. Le brevet français No. FR 2 936 710 délivré le 7 janvier 2011 au nom de la Demanderesse décrit l'administration de composés antiprolactiniques agonistes des récepteurs de la dopamine aux ruminants pendant la gestation en vue d'induire le tarissement de la lactation et favoriser l'involution mammaire.

La Demanderesse a maintenant découvert de manière surprenante que l'administration d'une composition vétérinaire comprenant au moins un composé antiprolactinique agoniste des récepteurs de la dopamine, et en particulier la cabergoline, à des ruminants lors de la mise en oeuvre de la période sèche et/ou pendant la période sèche, permettait de prévenir et/ou de réduire les effets délétères liés à la mise en oeuvre d'une période sèche raccourcie, tels que les maladies métaboliques et/ou les troubles de la reproduction, et permettait également d'améliorer le statut immunitaire et/ou les fonctions hépatiques des ruminants lors de la lactation suivante. De manière surprenante, l'administration des compositions vétérinaires selon la présente invention n'affecte toutefois pas la capacité de produire du lait et/ou la qualité du lait des ruminants traités.

### Résumé de l'invention

La présente invention concerne l'utilisation d'une composition vétérinaire comprenant de la cabergoline destinée à être administrée à un ruminant en traitement unique et/ou répété soit lors de la mise en oeuvre d'une période sèche raccourcie de 4 semaines, soit lors de la mise en oeuvre et pendant ladite période sèche raccourcie de 4 semaines, pour assurer ou maintenir la capacité de production du lait du ruminant lors de lactation suivante.

La présente divulgation concerne l'utilisation de compositions vétérinaires comprenant au moins un composé antiprolactinique agoniste des récepteurs de la dopamine administrées à des ruminants lors de la mise oeuvre et/ou pendant la période sèche. Les composés antiprolactiniques des compositions vétérinaires de la présente divulgation sont choisis parmi les composés agonistes des récepteurs de la dopamine issus de l'ergot de seigle ou bien parmi les composés agonistes non issus des dérivés de l'ergot de seigle.

Selon la divulgation ces compositions sont administrées en quantités thérapeutiques efficaces, lors de la mise en oeuvre de la période sèche et/ou pendant la période sèche des ruminants, en vue de prévenir et/ou de réduire les effets délétères liés à la mise en oeuvre d'une période sèche raccourcie chez les ruminants. Les effets délétères visés par ces traitements comprennent notamment les maladies métaboliques ainsi que les troubles de la reproduction. L'administration des compositions vétérinaires selon la présente divulgation permet par ailleurs d'améliorer le statut immunitaire et/ou les fonctions hépatiques des ruminants à la reprise de la lactation. Les compositions sont administrées aux ruminants au début et/ou pendant la période sèche, en traitement unique et/ou en traitement répété. L'administration conjointe des compositions vétérinaires à la réduction de la durée de la période sèche n'affecte pas la capacité de production et/ou la qualité du lait du ruminant lors de la lactation suivante.

La présente divulgation concerne également une méthode de traitement et/ou de prévention des effets délétères liés à la mise en oeuvre d'une période sèche raccourcie chez les ruminants, tels que les maladies métaboliques et/ou les troubles de la reproduction, ainsi qu'une méthode visant à améliorer le statut immunitaire et/ou les fonctions hépatiques chez les ruminants, comprenant l'administration selon un schéma thérapeutique efficace des compositions vétérinaires aux ruminants lors de la mise oeuvre et/ou pendant la période sèche.

### Brève description des Figures

**Figure 1** **:** est un graphe montrant le cycle annuel de la vache sur un cycle de 12 mois.
**Figures 2A et 2B** : sont des graphes montrant le suivi de la production de lait 60 jours post vêlage (2A), et la différence relative avec la production historique (305 jours de la lactation précédente) (2B), pour les groupes traités C646-1 et C646-2.
**Figure 3** **:** est un graphe illustrant l'induction du transcrit ARNm correspondant à la forme longue du récepteur de prolactine pendant la période sèche et jusqu'à 90 jours post vêlage dans le groupe des vaches ayant reçu une injection de cabergoline (C646-1 et C646-2).

### Description détaillée de l'invention

L'objet de la présente invention est tel que défini dans les revendications 1 à 4.

Le présent document décrit une composition vétérinaire comprenant au moins un composé antiprolactinique agoniste des récepteurs de la dopamine administrée en traitement unique et/ou en traitement répété lors de la mise oeuvre et/ou pendant la période sèche, pour son utilisation dans la prévention et/ou la diminution des effets délétères liés à la mise en oeuvre d'une période sèche raccourcie chez les ruminants.

Le présent document décrit également une composition vétérinaire comprenant au moins un composé antiprolactinique agoniste des récepteurs de la dopamine administrées en traitement unique et/ou en traitement répété lors de la mise en oeuvre et/ou pendant la période sèche, pour son utilisation dans la prévention et/ou la diminution des maladies métaboliques et/ou des troubles de la reproduction des ruminants à la reprise de la lactation.

Lorsque lesdites compositions sont administrées aux ruminants lors de la mise en oeuvre et/ou pendant la période sèche, celles-ci permettent d'améliorer le statut immunitaire ainsi que les fonctions hépatiques des ruminants à la reprise de la lactation, sans toutefois affecter la capacité à produire du lait ou sur la qualité du lait lors de la lactation suivante.

Le présent document décrit également une méthode de traitement et/ou de prévention des effets délétères liés à la mise en oeuvre d'une période sèche raccourcie chez les ruminants, tels que les maladies métaboliques et/ou les troubles de la reproduction, ainsi qu'une méthode visant à améliorer le statut immunitaire et/ou les fonctions hépatiques chez les ruminants, comprenant l'administration selon un schéma thérapeutique efficace des compositions vétérinaires aux ruminants lors de la mise en oeuvre et/ou pendant la période sèche. Cette méthode n'affecte pas la capacité de production et/ou la qualité du lait du ruminant.

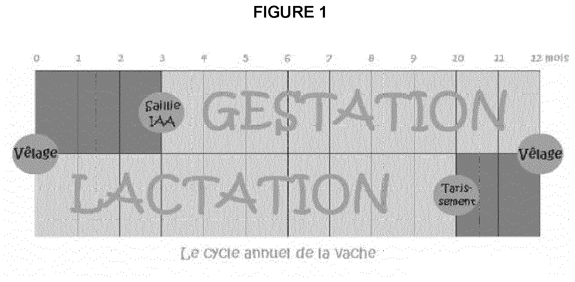

De préférence, lesdites compositions vétérinaires sont administrées aux ruminants tels que la vache laitière, lors de la mise en oeuvre de la période sèche, tel que cela est montré à la Figure 1.

Les compositions antiprolactiniques vétérinaires ainsi que les méthodes selon la présente divulgation sont attractives pour l'éleveur puisque ce dernier peut, en les utilisant, diminuer la durée de la période sèche qui se traduira par un accroissement de la période de production laitière, une gestion du troupeau simplifiée ainsi qu'une économie sur les coûts de traitements des maladies à la reprise de la lactation.

Comme cela est démontré dans les exemples ci-dessous, un traitement unique et/ou répété peut être administré aux ruminants lors de la mise oeuvre et/ou pendant la période sèche, en vue de prévenir et/ou de réduire les maladies métaboliques et les troubles de la reproduction, et améliorer le statut immunitaire et/ou les fonctions hépatiques des ruminants à la reprise de la lactation. A titre d'exemple, les compositions vétérinaires peuvent être administrées chez la vache laitière au début de la période sèche ou pendant cette période sèche.

Les périodes sèches conventionnelles sont généralement de 8 semaines. Par conséquent, selon la présente invention, on entend par période sèche raccourcie, une période sèche qui est réduite d' 1 semaine, ou de 2 semaines, ou de 3 semaines, ou encore de 4 semaines. De préférence, la période sèche raccourcie est réduite par rapport aux périodes sèches conventionnelles d'au plus 4 semaines, et de manière encore plus préférentielle, elle est réduite de 2 à 4 semaines. Par conséquent, selon la présente invention, la période sèche conventionnelle de 8 semaines est réduite à une période sèche raccourcie dont la durée est de préférence comprise entre 4 à 6 semaines.

L'administration des compositions selon le présent document, permet ainsi:
(i) la prévention et/ou la réduction des maladies métaboliques ;
(ii) la prévention et/ou la réduction des troubles de la reproduction ;
(iii) l'amélioration du statut immunitaire et des fonctions hépatiques à la reprise de la lactation;
(iv) une meilleure santé du nouveau-né ;
(v) l'absence d'effets sur la capacité de produire du lait et/ou sur la qualité du lait lors de la lactation suivante;
(vi) l'allongement de la période de production de la lactation en cours ; et
(vii) une meilleure gestion des programmes alimentaires pendant la période sèche.

Les composés antiprolactiniques sont choisis parmi les composés agonistes des récepteurs de la dopamine issus de l'ergot de seigle et/ou parmi les composés agonistes non issus des dérivés de l'ergot de seigle.

La prolactine est une hormone hypophysaire qui a un effet mammotrope et lactogénique c'est-à-dire qu'elle active la croissance des glandes mammaires et la sécrétion du lait. La libération de la prolactine est sous influence stimulatrice de la prolactolibérine et inhibitrice de la dopamine. L'action inhibitrice de la dopamine au niveau de l'hypophyse est médiée par des récepteurs post-synaptiques de la dopamine, tels que notamment le récepteur D2.

Les composés sont dits antiprolactiniques lorsqu'ils inhibent la libération de la prolactine. Les composés dopaminergiques sont des agonistes des récepteurs de la dopamine, susceptibles de se lier aux récepteurs de la dopamine présents notamment au niveau des cellules sécrétrices de la prolactine de l'antéhypophyse afin d'inhiber la sécrétion de prolactine.

De préférence, ces composés antiprolactiniques dopaminergiques se lient aux récepteurs de la dopamine. De manière encore plus préférentielle, ces composés se lient de manière spécifique aux récepteurs D2 de la dopamine. Les composés antiprolactiniques dopaminergiques agissent en stimulant la libération de dopamine dans l'hypothalamus, aboutissant ainsi à une inhibition de la sécrétion de prolactine.

L'hormone prolactine se lie sur des récepteurs membranaires (RPRL) présents sur les cellules des tissus notamment sur les glandes mammaires, le système immunitaire, et le tissu hépatique. Les RPRL comportent un domaine extracellulaire interagissant avec l'hormone, un domaine transmembranaire, et un domaine cytoplasmique impliqué dans la transmission du signal de la prolactine. La transmission du signal se fait par la translocation transmembranaire des tyrosines kinases associées. Il existe plusieurs isoformes du récepteur de la prolactine qui comprennent tous un domaine extracellulaire similaire, mais qui différent par la longueur de leur domaine transmembranaire. Dans l'espèce bovine, il a notamment été décrit une forme longue et une forme courte du récepteur. La prolactine se lie à ces différents récepteurs avec une affinité similaire, mais la translocation transmembranaire est favorisée par la présence et la quantité des formes longues. La co-expression des différentes isoformes de récepteurs de la prolactine (forme longue versus forme courte) pourrait moduler le signal cellulaire due à la prolactine.

Ces composés antiprolactiniques agonistes des récepteurs de dopamine peuvent être choisis parmi des composés issus de l'ergot de seigle et/ou leurs dérivés. Ces dérivés de l'ergoline sont bien connus dans le domaine public et présentent la structure générale suivante :

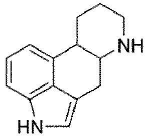

Parmi ces composés antiprolactiniques agonistes dérivés de l'ergot de seigle, on peut citer à titre d'exemples, la cabergoline, la métergoline, la lisurdine, la bromocriptine, l'ergométrine, ainsi que tous les dérivés de ces composés ayant une activité antiprolactinique.

La cabergoline utilisée dans la présente invention, dont le nom chimique est le N-[3-(Dimethylamino)propyl]-N-[(ethylamino)carbonyl]-6-(2-propenyl)-8g-ergoline-8-carboxamide, est un composé antiprolactinique agoniste spécifique des récepteurs D2 de la dopamine. Il est notamment décrit dans le brevet US 4,526,892. Sa formule chimique développée est la suivante :

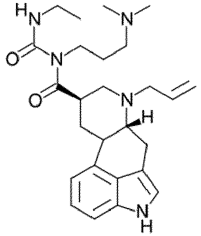

La cabergoline constitue le principe actif des médicaments humains commercialisés sous les dénominations Dostinex® et Cabaser®. Egalement, c'est le principe actif de base des compositions vétérinaires commercialisées sous la dénomination Galastop® destinées aux chiennes sujettes à des lactations de pseudo-gestation. Que ce soit le Dostinex® ou le Galastop®, ces compositions à base de cabergoline ne sont jamais administrées pendant la grossesse ou la gestation.

La métergoline est un composé synthétique connu sous le nom chimique benzyl ((6a*R*,9*S*,10a*R*)-4,7-dimethyl-4,6,6a,7,8,9,10,10a-octahydroindolo-[4,3-*fg*]quinolin-9-yl)méthyl carbamate. Il s'agit d'un composé antiprolactinique qui est susceptible de se lier à la fois aux récepteurs de la dopamine et aux récepteurs de la sérotonine 5HT et ainsi d'activer la libération de dopamine et l'inhibition de la sécrétion de prolactine. La métergoline a la formule chimique développée suivante :

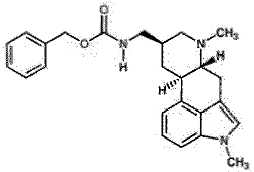

Le lisuride également connu sous le nom chimique 1,1-diéthyl-3-((6a*R*,9*S*)-7-méthyl-4,6,6a,7,8,9-hexahydroindolo[4,3-*fg*]quinolin-9-yl) urée présente la formule chimique développée est la suivante :

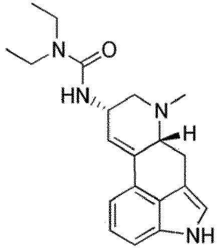

Il s'agit d'un composé agoniste dopaminergique inhibiteur de la prolactine. Il est commercialisé sous le nom d'Arolac® pour le traitement des aménorrhées et des hyperprolactinémies chez les patients humains.

La bromocriptine est connue sous le nom de bromo-2 ergocryptine et sous la dénomination chimique ergotaman-3',6',18-trione, 2-bromo-12'-hydroxy-2'-(1-methylethyl)-5'alpha-(2-methylpropyl). Sa formule chimique développée est la suivante :

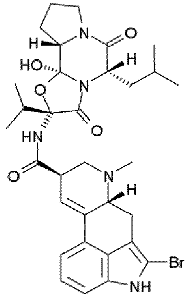

Des compositions comme le Parlodel® et le Bromo-Kin® utilisées notamment pour les traitements des tumeurs de la glande hypophysaire et des hyperprolactinémies, ou en gynécologie comme inhibiteur de la lactation après un accouchement comprennent une quantité thérapeutique de bromocriptine, un dérivé de l'ergot de seigle agoniste dopaminergique.

L'ergométrine ou ergonovine, encore dénommée acide d-lysergique beta-propanolamide peut également être incorporée dans les compositions vétérinaires en tant qu'agoniste antiprolactinique dopaminergique. Sa formule développée est la suivante :

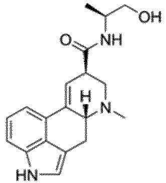

D'autres composés antiprolactiniques agonistes des récepteurs de la dopamine peuvent également être utilisés dans les compositions selon le présent document et être administrés aux ruminants particulièrement pendant la période de gestation. Ceux-ci peuvent être choisis parmi les composés agonistes dopaminergiques antiprolactiniques non issus de l'ergot de seigle. A titre d'exemples de ces composés, on peut citer le ropinirole, le pramipexole, la rotigotine, la quinagolide, ainsi que tous les dérivés de ces composés ayant une activité antiprolactinique.

Le ropinirole dont le nom chimique est le 4-(2-dipropylaminoethyl)-1,3-dihydroindol-2-one, agit en tant qu'agoniste des récepteurs D2 et D3 de la dopamine. Sa formule chimique développée est la suivante :

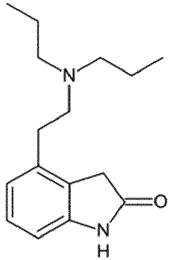

Le ropinirole entre dans la composition des produits Requip® et Ropark® prescrits en médecine humaine pour traiter la maladie de Parkinson.

Le pramipexole représente un autre agoniste dopaminergique qui se lie plus particulièrement aux récepteurs D2 et D3 de la dopamine. Son nom chimique est le (6*S*)-N⁶-propyl-4,5,6,7-tetrahydro-1,3-benzothiazole-2,6-diamine et sa formule développée est la suivante :

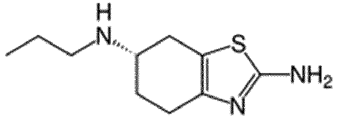

Le pramipexole est bien connu dans le domaine pharmaceutique humain puisqu'il est commercialisé sous les noms de Mirapex®, Mirapexin® ou Sifrol® pour le traitement de la maladie de Parkinson et du syndrome des jambes sans repos.

La rotigotine qui est également connue sous le dénomination chimique de 6-(propyl-(2-thiophen-2-ylethyl)amino)tetralin-1-ol. Sa formule chimique développée est comme suit :

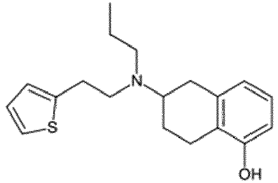

La rotigotine a été récemment approuvée pour le traitement de la maladie de Parkinson en administration transdermique sous forme de patch sous la désignation Neupro®.

La quinagolide ou (3*R*,4a*R*,10a*S*)-3-(diethylsulfamoylamino)-6-hydroxy-1-propyl-3,4,4a,5,10,10a-hexahydro-2*H-*benzo[g]quinoline est également un inhibiteur de la sécrétion de prolactine. Il s'agit d'un agoniste spécifique des récepteurs D2 de la dopamine. Sa formule chimique développée est la suivante :

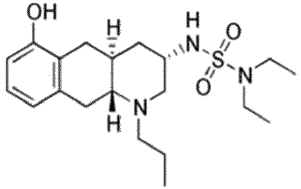

La quinagolide est commercialisée sous la désignation commerciale Norprolac® (Ferring Pharmaceuticals) pour le traitement des macroprolactinomes ou des hyperprolactinémies.

Les compositions vétérinaires peuvent être administrées en traitement unique et/ou répété lors de la mise oeuvre et/ou pendant la période sèche.

Les quantités ou doses thérapeutiques efficaces sont susceptibles de varier en fonction des ruminants à traiter et selon le mode d'administration des compositions. Les dosages, encore appelés schémas thérapeutiques, peuvent facilement être déterminés par des essais systématiques sur la base des exemples ci-dessous et sont à la portée de l'homme du métier. A titre d'exemples, les doses thérapeutiques efficaces selon la présente invention sont comprises entre 1 et 100 mcg/kg, ou entre 5 et 50 mcg/kg, et de préférence d'environ 8 à 10 mcg/kg.

Dans le cas des vaches laitières, il est possible d'administrer les compositions selon l'invention lors de la mise oeuvre et/ou pendant la période sèche, par exemple après les 11 premiers mois du cycle annuel de la vache (Figure 1).

Selon l'invention, on entend par ruminants, les mammifères herbivores tels que par exemple les bovins, les ovins, les caprins, les camélidés, ou les bovidés. Les compositions selon l'invention sont administrées aux mammifères ruminants producteurs de lait, tels que de préférence les vaches et les brebis laitières.

Les compositions vétérinaires peuvent être administrées selon toutes voies d'administration bien connues dans le domaine et adaptées au traitement de chacun des animaux. De préférence, elles sont administrées par voies cutanée, orale, intramammaire et parentérale. De manière encore plus préférentielle, elles sont administrées par voie parentérale, et notamment par injection intramusculaire ou sous-cutanée. Elles peuvent donc être sous la forme d'une solution ou d'une suspension liquide orale, intramammaire ou injectable, ou sous la forme solide ou semi-solide, de poudres, de comprimés, de capsules, de granules, de dragées, de gélules, de sprays, de cachets, de pilules, de tablettes, ou de pâtes.

De manière avantageuse, les compositions vétérinaires peuvent être administrées en une seule ou plusieurs doses injectables.

Selon les formulations des compositions utilisées, elles peuvent comprendre en outre des ingrédients conventionnellement utilisés en pharmacie pour la préparation des formulations liquides ou solides pour une administration par voie orale, intramammaire ou parentérale. Par ailleurs, dans le cas de formulations orales, elles peuvent être administrées directement aux ruminants ou mélangées à la nourriture.

Egalement, les compositions selon l'invention peuvent comprendre selon le type de formulations, un solvant, un agent d'écoulement, un lubrifiant et tout excipient de masse convenable, tel que le lactose, la cellulose ou les amidons. Comme lubrifiant, on peut utiliser l'acide stéarique, le stéarate de magnésium, la L-leucine ou, par exemple, le tribéhénate de glycérol. Comme agent de désintégration, on peut utiliser le carboxyméthylamidone sodique, la carboxyméthylcellulose sodique réticulée, ou la polyvinylpyrrolidone réticulée. Comme agent d'écoulement, on peut utiliser la silice pure ou le dioxyde de silicium colloïdal. Les formes orales solides peuvent être sous formes de comprimés recouverts d'un enrobage.

Les préparations injectables sont préparées par mélange de quantités thérapeutiques efficaces d'au moins un composé antiprolactinique tel que précédemment décrit avec un solvant, un régulateur de pH, un agent tampon, un agent de mise en suspension, un agent de solubilisation, un stabilisant, un agent de tonicité et/ou un conservateur, et par transformation du mélange en une injection sous-cutanée ou intramusculaire selon un procédé classique. Comme solvant, on peut citer le dimethylsulfoxyde (DMSO), les solvants huileux tels que les triglycérides à chaine moyenne en C₈-C₁₀, ou un mélange d'acide caprique, d'acide caprylique et de triglycérides, tels que ceux qui sont commercialisés sous la désignation Mygliol®812. Le cas échéant, les préparations injectables peuvent être lyophilisées selon un procédé classique. Des exemples d'agents de mise en suspension englobent la méthylcellulose, le polysorbate 80, l'hydroxyéthylcellulose, la gomme de xanthane, la carboxyméthycellulose sodique et le monolaurate de sorbitane polyéthoxylé. Des exemples d'agent de solubilisation englobent l'huile de ricin solidifiée par du polyoxyéthylène, le polysorbate 80, le nicotinamide, le monolaurate de sorbitane polyéthoxylé, le macrogol et l'ester éthylique d'acide gras d'huile de ricin. En outre, le stabilisant englobe le sulfite de sodium, le métalsulfite de sodium et l'éther, tandis que le conservateur englobe le p-hydroxybenzoate de méthyle, le p-hydroxybenzoate d'éthyle, l'acide sorbique, l'alcool benzylique, le phénol, le crésol et le chlorocrésol. Un exemple d'agent de tonicité est le mannitol. Lors de la préparation des solutions ou suspensions injectables, il est souhaitable de veiller à ce qu'elles soient isotoniques au sang.

Le présent document décrit en outre un kit à usage vétérinaire destiné à prévenir et/ou réduire les effets délétères liés à la mise en oeuvre d'une période sèche raccourcie chez les ruminants, tels que les maladies métaboliques et/ou les troubles de la reproduction, et à améliorer le statut immunitaire et/ou les fonctions hépatiques à la reprise de la lactation des ruminants. Les kits comportent au moins un compartiment pour un conditionnement éventuellement stérile comprenant une quantité thérapeutique efficace d'au moins un composé antiprolactinique agoniste des récepteurs de la dopamine tel que précédemment décrit, pour une administration aux ruminants. Le kit comprend les moyens permettant l'administration des compositions par voie cutanée, orale, intramammaire ou parentérale ainsi qu'une fiche d'instructions concernant le mode d'administration des compositions ou médicaments vétérinaires.

### EXEMPLES

### Exemple 1 : Préparation de compositions vétérinaires à base de cabergoline

Préparation de 9 litres d'une préparation pour solution injectable contenant 500 mcg/ml de cabergoline.

### Formule :

- Principe actif : 4,53 g de cabergoline (titrant 100 %)
- Excipient : triglycérides à chaîne moyenne q. s. 9 I.
   **Etape 1 :** 4,53 g de cabergoline et 1,5 kg de triglycérides à chaîne moyenne ont été mesurés dans un récipient de capacité adéquate, puis le mélange a été agité à l'aide d'un agitateur magnétique (500 tours minute) pendant au moins 60 minutes pour une dissolution complète.
   **Etape 2 :** dans un récipient sec, 5 kg de triglycérides à chaîne moyenne ont été mesurés, le flux d'azote et le mélangeur ont été mis en route, puis la cabergoline en solution concentrée, obtenue à l'étape 1, a été ajoutée dans le récipient. L'agitation a été maintenue pendant 30 minutes puis le volume a été porté au volume final de 9 litres en ajoutant des triglycérides à chaîne moyenne. L'agitation a été encore maintenue 30 minutes puis la solution a été filtrée sous azote pressurisé au travers d'une cartouche calibrée à 0,45 microns. Le filtrat a été collecté dans un bidon approprié préalablement désinfecté à la vapeur et séché avec un flux d'azote. La solution obtenue a été stérilisée à 121 °C pendant 15 minutes, puis elle a été répartie dans des flacons stériles et apyrogènes fermés avec des bouchons caoutchouc et des capsules en aluminium, lavés et passés à l'autoclave.

### Exemple 2 : Démonstration de l'absence d'effets délétères sur la production de lait chez la vache laitière traitées avec les compositions vétérinaires à base de cabergoline

Cette étude a pour objectif d'évaluer l'efficacité des compositions vétérinaires à base de carbergoline chez des vaches laitières, primipares (n= 17) ou multipares (n= 54) de race Prim'Holstein, en lactation et gestantes de 8 mois lors de l'administration, de telle sorte que la durée de leur période sèche soit réduite à 1 mois. Les vaches laitières sont gardées dans une étable selon les conditions courantes d'élevage.
L'expérimentation comprend 3 groupes de vaches:
- le groupe contrôle (n=24 vaches) qui reçoit deux administrations de placébo à D0 (début de la période sèche par le tarissement) et à D10 (10 jours suivant D0);
- le groupe C646-1 (n= 23 vaches) qui reçoit une dose unique de composition vétérinaire à base de carbergoline à D0;
- le groupe C646-2 (n=24 vaches) qui reçoit deux doses de composition vétérinaire à base de carbergoline à D0 puis à D10 ;

La composition vétérinaire à base de carbergoline est une solution huileuse injectable de cabergoline telle que décrite dans l'Exemple 1. Elle est administrée par injection intramusculaire dans le cou à une dose de 5.6mg/vache soit environ 8 mcg/kg.

Les trois groupes de vaches traitées sont inclus dans l'étude à environ 45 jours avant vêlage (D-15) et sont suivis pendant les trois périodes suivantes :
- à la fin de la lactation (n-1) précédent D0 :
- pendant la période sèche d'1 mois : depuis le tarissement du lait (D0) jusqu'au vêlage (C0 correspondant au alentour de D30),
- à la lactation (n) pendant les 3 premiers mois suivants le vêlage (C0).

Les périodes sèches effectives des trois groupes sont listées dans le tableau 1 ci-dessous :

**Tableau 1**

| Groupes | Périodes Sèches effectives (écart type) |
|---|---|
| Placébo | 31 jours (5,0) |
| C646-1 | 27 jours (4,8) |
| C646-2 | 29 jours (5,4) |

Les productions de lait 3 mois après vêlage (C0) sont obtenues pour chaque vache traitée et sont comparés entre elles en prenant compte les productions obtenues lors de la lactation précédente (n-1).

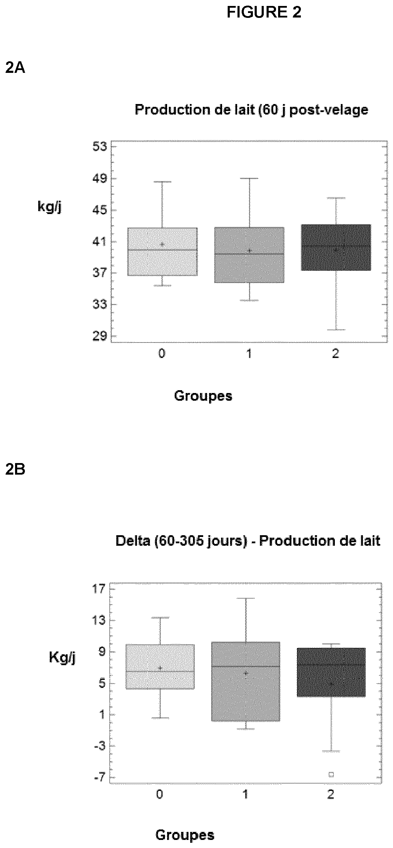

Le suivi de la production du lait 60 jours post vêlage (Figure 2A) ainsi que la différence relative avec la production historique (305 jours de la lactation précédente), (Figure 2B) montre que la durée de la période sèche suite aux traitements avec les compositions vétérinaires selon la présente demande n'entraîne pas d'effets délétères dans la capacité à produire du lait des vaches laitières traitées.

### Exemple 3: Etude d'efficacité chez la vache laitière de compositions vétérinaires

### à base de cabergoline

Les trois groupes de vaches traitées tels que décrites dans l'Exemple 2 font l'objet des examens et prélèvements suivants :
- Surveillance quotidienne clinique générale:
   ∘ Survenue de Maladies (métaboliques, infectieuses...) ;
   ∘ Tous les signes anormaux sont notés, tels que notamment les avortements, ainsi que toutes difficultés lors du vêlage. Les animaux sont suivis jusqu'à la mise bas afin d'évaluer l'état de santé des veaux nouveau-nés.
- Prélèvement sanguins à D0, D10, D20, C0 (Vêlage = D30), C10, C30 et C90.
   ∘ Ces prélèvements nous permettent de doser par PCR notamment les récepteurs à la prolactine considérés comme marqueurs de la santé des animaux,
- Dosage du colostrum (IgG) à C0
- Comptage des cellules somatiques dans le lait produit à C30, C60 et C90.

Les résultats observés font apparaître que la réduction de la durée de la période sèche se traduit par notamment par une expression accrue des récepteurs de forme longue à la prolactine dans les groupes C646-1 et C646-2 par rapport au groupe placébo.

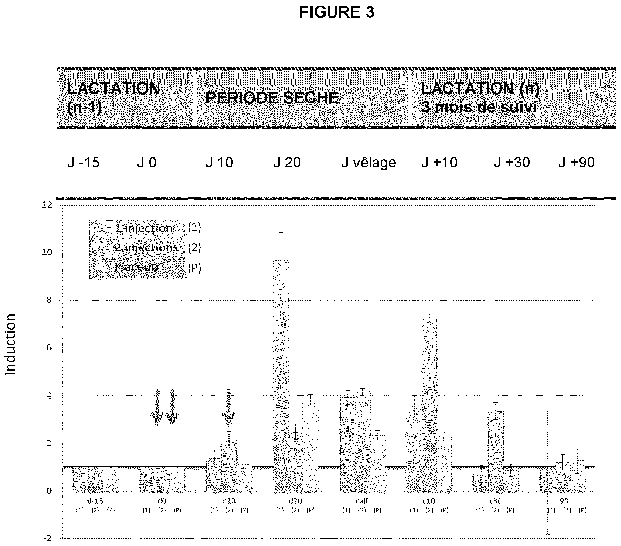

La Figure 3 montre en effet une induction de l'expression de l'isoforme longue du récepteur de prolactine pendant la période sèche et à 10 jours puis 30 jours post vêlage et ceci dans le groupe des vaches ayant reçu une injection de composition vétérinaire selon la présente invention.

## Revendications

1. Utilisation d'une composition vétérinaire comprenant de la cabergoline destinée à être administrée à un ruminant en traitement unique et/ou répété soit lors de la mise en oeuvre d'une période sèche raccourcie de 4 semaines, soit lors de la mise en oeuvre et pendant ladite période sèche raccourcie de 4 semaines, pour assurer ou maintenir la capacité de production du lait du ruminant lors de lactation suivante.

2. Utilisation selon la revendication 1, dans laquelle la composition est destinée à être administrée à des doses d'environ 8 à 10 mcg/kg.

3. Utilisation selon la revendication 1 ou 2, **caractérisée en ce que** les ruminants sont des mammifères herbivores choisis parmi les bovins, les ovins, les caprins, les camélidés, ou les bovidés, en particulier une vache laitière.

4. Utilisation selon l'une quelconque des revendications précédentes, **caractérisée en ce que** ladite composition est administrée par voie parentérale, intramammaire, cutanée ou orale.

## Patentansprüche

1. Verwendung einer tiermedizinischen Zusammensetzung, die Cabergolin enthält und an einen Wiederkäuer in einer einzigen Behandlung und/oder wiederholten Behandlung entweder bei der Implementierung einer verkürzten Trockenphase von 4 Wochen oder bei der Implementierung und während besagter verkürzter Trockenphase von 4 Wochen verabreicht werden soll, um die Fähigkeit des Wiederkäuers zur Milchproduktion bei der nachfolgenden Laktation sicherzustellen oder aufrechtzuerhalten.

2. Verwendung gemäß Anspruch 1, wobei die Zusammensetzung mit Dosen von ungefähr 8 bis 10 µg/kg verabreicht werden soll.

3. Verwendung gemäß Anspruch 1 oder 2, **dadurch gekennzeichnet, dass** die Wiederkäuer herbivore Säugetiere, ausgewählt aus Rindern, Schafen, Ziegen, Kamelen oder Horntieren, insbesondere Milchkühe sind.

4. Verwendung gemäß einem der vorhergehenden Ansprüche, **dadurch gekennzeichnet, dass** besagte Zusammensetzung parenteral, intramammär, kutan oder oral verabreicht wird.

## Claims

1. Use of a veterinary composition comprising cabergoline to be administered to a ruminant in a single treatment and/or in repeated treatments either at the time of the implementation of a shortened dry period of 4 weeks or at the time of the implementation of or during the shortened dry period of 4 weeks, for ensuring or maintaining milk-producing ability of the ruminant during the subsequent lactation.

2. Use according to claim 1, wherein the composition is to be administered at doses from about 8 to 10 µg/kg.

3. Use according to claim 1 or 2, wherein the ruminants are herbivorous mammals selected in the group consisting of bovines, sheep, caprines, camelids and bovids, more preferably dairy cows.

4. Use according to anyone of the preceding claims, wherein said composition is administered by parenteral, intramammary, cutaneous or oral route.
